# EUROPEAN PATENT APPLICATION

(11) **EP 1 550 403 A1**
(43) Date of publication of application: **06.07.2005**
(21) Application number: 03758717.7
(22) Date of filing: 08.10.2003
(51) Int. Cl.: A61B 8/14

(54) **ULTRASONIC DIAGNOSING DEVICE**

(30) Priority: 09.10.2002 JP 2002296634; 28.10.2002 JP 2002313121
(71) Applicant: MATSUSHITA ELECTRIC INDUSTRIAL CO., LTD., Kadoma-shi, Osaka 571-8501 (JP)
(72) Inventor: NISHIGAKI, Morio, Fujisawa-shi, Kanagawa 251-0028 (JP); SATOU, Toshiharu, Kawasaki-shi, Kanagawa 214-0036 (JP); HAGIWARA, Hisashi, Yokohama-shi, Kanagawa 227-0066 (JP); TANNAKA, Yoshinao, Aikou-gun, Kanagawa 243-0301 (JP)
(74) Representative: Hössle Kudlek & Partner
(86) International application number: PCT/JP2003/012896
(87) International publication number: WO 2004/032747

(57) **Abstract**

An ultrasonic diagnostic apparatus that can execute the positioning of a probe and an inspection sample easily and accurately at an excellent reproducibility is disclosed. This is the ultrasonic diagnostic apparatus that includes; a probe for transmitting an ultrasonic wave into a living body and receives a reflection wave from an inspection sample inside the living body; an image generator for generating a fault image of the inspection sample in accordance with a signal received by the probe; and an image display for displaying the fault image. The probe has a first transducer element array 1 and a second transducer element array 2, and these transducer element arrays are arranged such that array directions intersect each other. Moreover, the image generator and the image display generate and display a first fault image corresponding to a signal received by the first transducer element array and a second fault image corresponding to a signal received by the second transducer element array.

## Description

### TECHNICAL FIELD

The present invention relates to an ultrasonic diagnostic apparatus for performing an ultrasonic transmission/reception through a transducer element array and obtaining information of a body.

The present invention relates to an ultrasonic diagnostic apparatus for obtaining information of a straight organ, for example, such as a blood vessel.

### BACKGROUND ART

An ultrasonic diagnostic apparatus performs an ultrasonic transmission/reception on a living body and consequently obtains two-dimensional information inside the living body, and this is used in various medical fields. Such an ultrasonic diagnostic apparatus, there are known: a B-mode displaying apparatus for using amplitude information and obtaining a fault image of an inspection sample; a Doppler blood flow meter for using a fact that a phase of a reflection wave of moving blood is changed with aged deterioration; and a color flow blood flow image apparatus; and the like. Also, in recent years, an ultrasonic diagnostic apparatus for obtaining information not only for a relatively fast motion such as a flow of blood but also for a relatively slow motion such as a motion of organ is proposed (for example, Japanese Patent Application Publication (KOKOKU) No.H7-67451). The ultrasonic diagnostic apparatus includes a probe for transmitting an ultrasonic wave to an inspection sample inside a living body and receiving a reception wave from the inspection sample. This probe has a transducer element array, and consequently repeats an ultrasonic transmission/reception inside the body and obtains the two-dimensional information of the body.

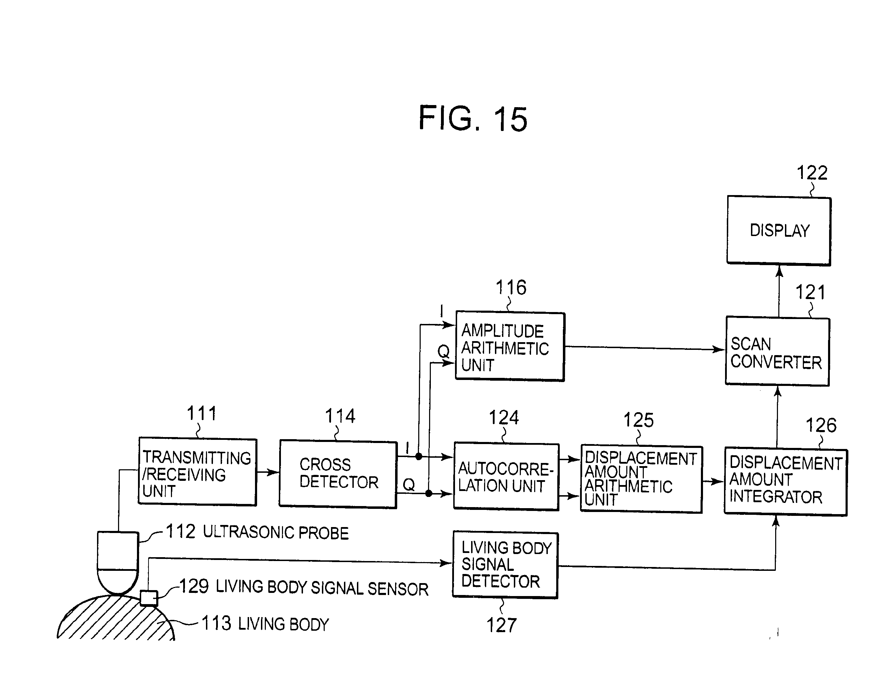

Fig. 15 is a block diagram of the apparatus noted in the foregoing gazette. In Fig. 15, an electronic pulse of a high voltage outputted from a transmitting/receiving unit 111 is converted into an ultrasonic signal in an ultrasonic probe 112 and transmitted in a direction where information of a living body 113 is desired to be obtained. The ultrasonic pulse is reflected in an organ (or a blood vessel or the like), in which the information inside the living body 113 is desired to be obtained, and received by the ultrasonic probe 112 and passed through the transmitting/receiving unit 111 and detected by an cross detector 114 by using a reference signal of a frequency approximately equal to a transmission frequency, and two signals of I, Q are outputted.

The two signals of I, Q are inputted to an amplitude arithmetic unit 116 and converted into amplitude information, and this signal is used for the B-mode displaying. The signals of I, Q are also inputted to an autocorrelation unit 124. The autocorrelation unit 124 correlates the equal depths of the signals that are transmitted and received in the same direction two times and consequently determines a rotation amount of a phase. The rotation amount of the phase is proportional to the movement amount of the organ. It is a displacement amount arithmetic unit 125 that carries out this computation. A displacement amount computed by the displacement amount arithmetic unit 125 is integrated by a displacement amount integrator 126. Consequently, by totaling the micro motions from a certain time, it is possible to determine the position to which the organ moves.

The B-mode image determined by the amplitude arithmetic unit 116 and the displacement amount determined by the displacement amount integrator 126 are displayed through a scan converter 121 on a display 122. Also, a living body signal sensor 129 and a living body signal detector 127 detect the information of the living body 113, for example, a heartbeat or the like, and determine a standard position in the displacement amount integrator 126. While a direction where the ultrasonic pulse is emitted to the living body 113 is sequentially changed, the foregoing operations are carried out, which enables the motion to be displayed as a two-dimensional image.

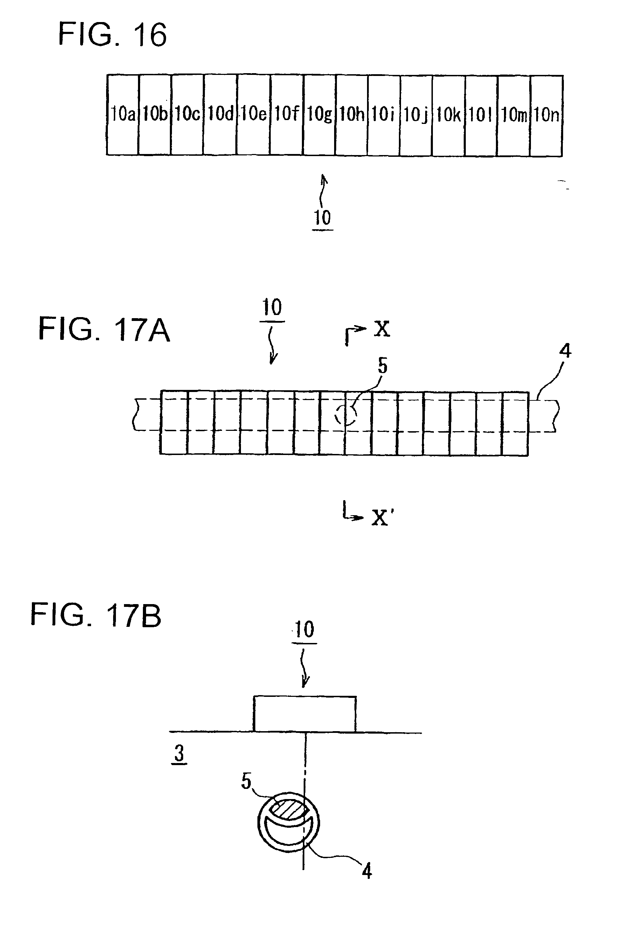

Fig. 16 is a diagrammatic view showing one example of the probe constituting the conventional ultrasonic diagnostic apparatus. This probe includes a transducer element array 10 in which a plurality of transducer elements 10ₐ to 10ₙ are arrayed in one direction.

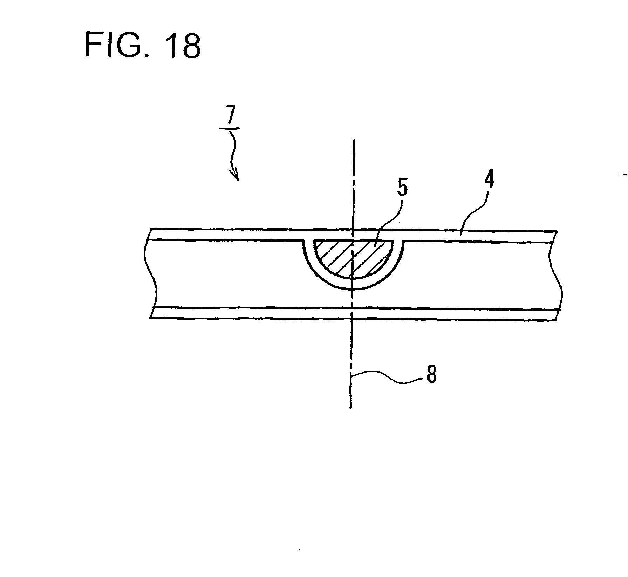

However, the foregoing ultrasonic diagnostic apparatus has a problem that the alignment of the probe is difficult in measuring. This problem is explained by exemplifying a case that a test article is an atheroma inside the blood vessel, with reference to Fig. 17A, Fig. 17B and Fig. 18.

Fig. 17A is a diagrammatic view showing a position relation between the probe and the inspection sample in the conventional ultrasonic diagnostic apparatus and corresponds to a top view, and Fig. 17B corresponds to its X-X' section view. Fig. 18 is a diagrammatic view showing one example a screen displaying at this time.

In measuring, the probe is arranged as shown in Fig. 17A, in which an array direction of the transducer element array 10 and a blood flow direction of a blood vessel 4 agrees and an atheroma 5 is located under the transducer element array 10. At this time, as shown in Fig. 18, on a displaying screen, together with a fault layer 7 of the blood vessel 4, a central position of the transducer element array 10, in short, a guide line 8 indicating a transmission direction of an ultrasonic beam 6 is displayed. The positioning of the probe is executed by moving the probe so that the guide line 8 agrees with the position of the atheroma 5 of the fault layer 7. However, even if the guide line 8 and the position of the atheroma 5 agree on the displaying screen, actually, as shown in Fig. 17B, there may be a possibility that the central position of the transducer element array 10 and the position of the atheroma 5 disagree with respect to a lateral section direction of the blood vessel 4 (a vertical direction to an axially central direction of the blood vessel 4). If such positional disagreement occurs, it is difficult to accurately measure the inner state of the atheroma 5 at an excellently reproducible manner. This is a problem that is similarly induced in inspection samples except the atheroma (for example, a tumor inside a liver, a polyp inside a gallbladder and the like).

In the conventional example, for example, when the blood vessel is observed as the straight organ, a problem that it is difficult to align the array direction of the ultrasonic probe 112 with the direction of the blood vessel is induced. Here, it is desired to position the ultrasonic probe 112 so that images appears such as a blood vessel wall and blood portion close to the ultrasonic probe 112 and a blood vessel wall away from the ultrasonic probe 112. However, this requires a skilled technique.

### DISCLOSURE OF THE INVENTION

The present invention has a first object to provide an ultrasonic diagnostic apparatus that can solve those problems and can carry out an observation by positioning to a straight organ such as a blood vessel and the like without any skilled technique and can obtain motion information of a high precision.

Also, the present invention has a second object to provide an ultrasonic diagnostic apparatus that can position a probe to a position of an inspection sample accurately at an excellent reproducibility.

In order to attain the foregoing first object, the present invention is designed such that it includes a plurality of transducer element arrays joined at any angle; and a means for displaying images respectively obtained from each of the plurality of transducer element arrays, wherein the transducer element array is configured by arraying a plurality of transducer elements in a parallel state.

That is, as one example, array directions of the plurality of transducer element arrays are arranged so as to be orthogonal. Concretely, for example, two transducer element arrays are arranged in a T-shaped type. Also, two transducer element arrays are arranged in a cross-shaped type. Also, three transducer element arrays are arranged in an H-shaped type.

With the foregoing configuration, after a center of one of the plurality of transducer element arrays is positioned to the straight organ, the other array direction can be positioned to the direction of the straight organ. Thus, the observation can be carried out by positioning to the straight organ such as the blood vessel and the like without any skilled technique, and the motion information of the high precision can be obtained.

In order to attain the foregoing second object, an ultrasonic diagnostic apparatus is characterized by including: a probe for transmitting an ultrasonic wave into a living body and receiving a reflection wave from an inspection sample inside the living body; an image generator for generating a fault image of the inspection sample in accordance with a signal received by the probe; and an image display for displaying the fault image,
wherein the probe has a first transducer element array and a second transducer element array which are arranged such that array directions of transducer elements intersect each other, and
the image generator and the image display generate and display a first fault image corresponding to a signal received by the first transducer element array and a second fault image corresponding to a signal received by the second transducer element array.

In the foregoing ultrasonic diagnostic apparatus, the probe has the plurality of transducer element arrays, and the plurality of fault images are displayed correspondingly to the respective transducer element arrays. Thus, since the positions of the probe and the inspection sample can be checked from a plurality of different directions, the position of the probe can be aligned with the position of the inspection sample easily and surely at the excellent reproducibility.

In the foregoing ultrasonic diagnostic apparatus, preferably, the image display displays a guide line that indicates positions of the first transducer element array and the second transducer element array, together with a fault image of an inspection sample. According to this preferred example, since the position of the probe can be easily checked on the fault image, the positioning of the probe can be executed further easily.

Also, in the foregoing ultrasonic diagnostic apparatus, preferably, the first transducer element array and the second transducer element array are arranged so as not to overlap with each other. The intersection between the first transducer element array and the second transducer element array requires the change in the shape of the transducer element array, such as the narrower widths of the mutual transducer elements in the intersection portion and the like. However, such a change may drop a reception sensitivity of the ultrasonic wave. However, according to this preferred example, such a problem can be avoided.

In this case, the first transducer element array can be designed so as to carry out a linear scanning. On the other hand, the second transducer element array can be designed so as to transmit and receive an ultrasonic wave that travels obliquely to a living body surface. Also, the second transducer element array may carry out a sector scanning.

Also, in the foregoing ultrasonic diagnostic apparatus, preferably, a width of the first transducer element array is adjusted so as to be small in a portion close to the second transducer element array.

As the inspection samples targeted by the foregoing ultrasonic diagnostic apparatus, for example, the organ and blood vessel inside the living body, and the atheroma existing inside the blood vessel, and the like are listed. In particular, knowing the situation of the atheroma is important for the arterial sclerosis diagnosis of a myocardial infarction, an angina and the like. However, the foregoing ultrasonic diagnostic apparatus is suitable for obtaining the information with regard to the situation of this atheroma.

### BRIEF DESCRIPTION OF THE DRAWINGS

Fig. 1A is an explanation view showing a probe that uses two transducer element arrays in a first embodiment of the present invention, and its positioning, and a top view showing arrangement directions of tow transducer element arrays A, B;
Fig. 1B is an explanation view showing the probe that uses the two transducer element arrays in the first embodiment of the present invention, and its positioning, and a top view showing a situation where the transducer element array B is used to position;
Fig. 1C is an explanation view showing the probe that uses the two transducer element arrays in the first embodiment of the present invention, and its positioning, and a right side view showing a situation that the positioning of the transducer element array B is completed;
Fig. 1D is an explanation view showing the probe that uses the two transducer element arrays in the first embodiment of the present invention, and its positioning, and a top view showing a situation where the transducer element array A is used to position;
Fig. 1E is an explanation view showing the probe that uses the two transducer element arrays in the first embodiment of the present invention, and its positioning, and a front view showing a situation that the positioning of the transducer element array A is completed;
Fig. 2 is a block diagram showing one example of an ultrasonic diagnostic apparatus in the first embodiment of the present invention;
Fig. 3 is a block diagram showing another example of the ultrasonic diagnostic apparatus in the first embodiment of the present invention;
Fig. 4A is an explanation view showing a probe that uses two transducer element arrays in a second embodiment of the present invention, and its positioning, and a top view showing arrangement directions of tow transducer element arrays A, B;
Fig. 4B is an explanation view showing the probe that uses the two transducer element arrays in the second embodiment of the present invention, and its positioning, and a top view showing a situation where the transducer element array B is used to position;
Fig. 4C is an explanation view showing the probe that uses the two transducer element arrays in the second embodiment of the present invention, and its positioning, and a right side view showing a situation that the positioning of the transducer element array B is completed;
Fig. 4D is an explanation view showing the probe that uses the two transducer element arrays in the second embodiment of the present invention, and its positioning, and a top view showing a situation where the transducer element array A is used to position;
Fig. 4E is an explanation view showing the probe that uses the two transducer element arrays in the second embodiment of the present invention, and its positioning, and a front view showing a situation that the positioning of the transducer element array A is completed;
Fig. 5A is an explanation view showing a probe that uses three transducer element arrays in a third embodiment of the present invention, and its positioning, and a top view showing arrangement directions of three transducer element arrays A, B and C;
Fig. 5B is an explanation view showing the probe that uses the three transducer element arrays in the third embodiment of the present invention, and its positioning, and a top view showing a situation where the transducer element array B is used to position;
Fig. 5C is an explanation view showing the probe that uses the three transducer element arrays in the third embodiment of the present invention, and its positioning, and a right side view showing a situation that the positioning of the transducer element array B is completed;
Fig. 5D is an explanation view showing the probe that uses the three transducer element arrays in the third embodiment of the present invention, and its positioning, and a top view showing a situation where the transducer element array C is used to position;
Fig. 5E is an explanation view showing the probe that uses the three transducer element arrays in the third embodiment of the present invention, and its positioning, and a left side view showing a situation that the positioning of the transducer element array C is completed;
Fig. 5F is an explanation view showing the probe that uses the three transducer element arrays in the third embodiment of the present invention, and its positioning, and a front view showing a situation that the positioning of the transducer element array C is completed;
Fig. 6 is a configuration view explaining one example of the configuration of the ultrasonic diagnostic apparatus according to the present invention;
Fig. 7 is a diagrammatic view showing one example of a probe constituting an ultrasonic diagnostic apparatus according to a fourth embodiment of the present invention;
Fig. 8A is a diagrammatic view showing a position relation between the probe and an inspection sample when the ultrasonic diagnostic apparatus is used, and the diagrammatic view in a top surface direction;
Fig. 8B is a diagrammatic view showing the position relation between the probe and the inspection sample when the ultrasonic diagnostic apparatus is used, and the diagrammatic view in its II-II' section direction;
Fig. 8C is a diagrammatic view showing the position relation between the probe and the inspection sample when the ultrasonic diagnostic apparatus is used, and the diagrammatic view in the I - I ' section direction;
Fig. 9 is a view showing one example of a screen displaying in the ultrasonic diagnostic apparatus;
Fig. 10 is a diagrammatic view showing another one example of the probe when the ultrasonic diagnostic apparatus is used, and the diagrammatic view in the I-I' section direction of Fig. 8A;
Fig. 11 is a diagrammatic view showing one example of a probe constituting an ultrasonic diagnostic apparatus according to a fifth embodiment of the present invention;
Fig. 12 is a diagrammatic view showing a position relation between the probe and the inspection sample when the ultrasonic diagnostic apparatus is used, and the diagrammatic view in the IV-IV' section direction of Fig. 11;
Fig. 13 is a diagrammatic view showing one example of a probe constituting an ultrasonic diagnostic apparatus according to a sixth embodiment of the present invention;
Fig. 14 is a diagrammatic view showing a position relation between the probe and the inspection sample when the ultrasonic diagnostic apparatus is used, and the diagrammatic view in the V-V' section direction of Fig. 13;
Fig. 15 is a block diagram of an ultrasonic diagnostic apparatus in a conventional example;
Fig. 16 is a diagrammatic view explaining one example of a probe constituting the conventional ultrasonic diagnostic apparatus;
Fig. 17A is a diagrammatic view showing a position relation between the probe and an inspection sample when the conventional ultrasonic diagnostic apparatus is used, and the diagrammatic view in a top surface direction;
Fig. 17B is a diagrammatic view showing a position relation between the probe and the inspection sample when the conventional ultrasonic diagnostic apparatus is used, and the diagrammatic view in its X-X' section direction; and
Fig. 18 is a diagrammatic view showing one example of a screen displaying in the conventional ultrasonic diagnostic apparatus.

### BEST MODE FOR CARRYING OUT THE INVENTION

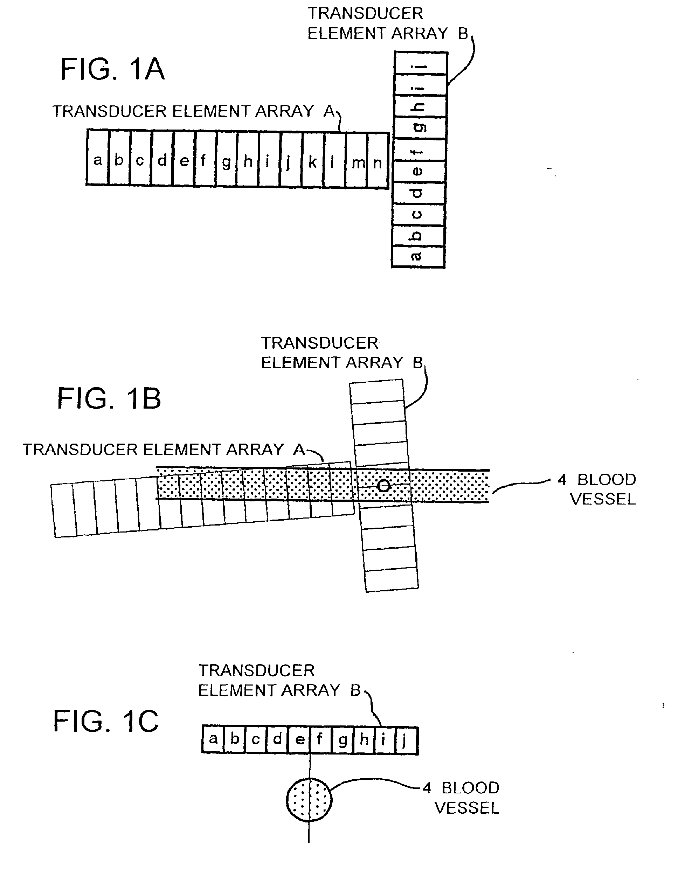

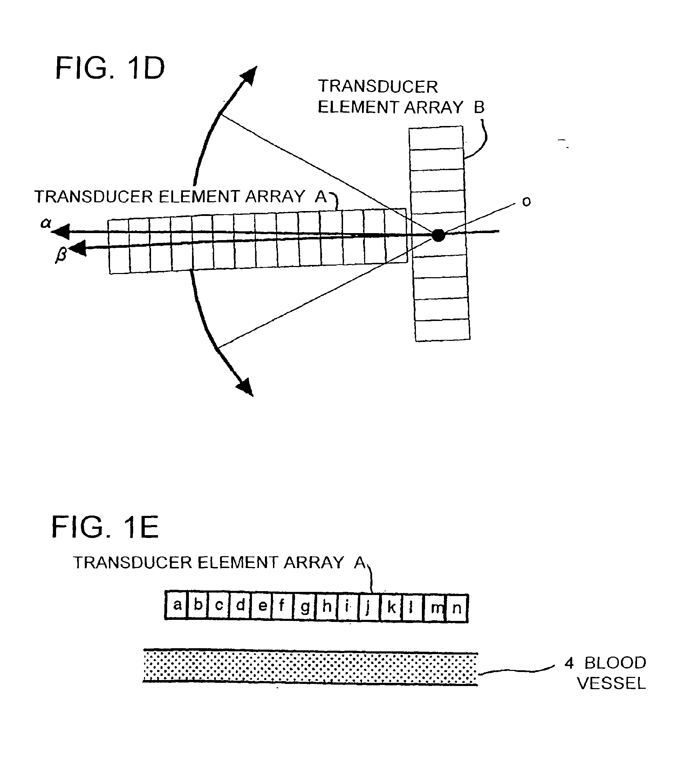

Embodiments of the present invention will be described below with reference to Fig. 1 to Fig. 14.

### <First Embodiment>

Figs. 1A to 1E are explanation views when inspection regions of a plurality of transducer element arrays in the first embodiment of the present invention are positioned to a blood vessel. Fig. 1A is a top view showing the arrangement directions of two transducer element arrays A, B. The transducer element array A is configured such that transducer elements a to n are straightly arrayed, and the transducer element array B is configured such that transducer elements a to j are straightly arrayed. The transducer element array A and the transducer element array B are arranged in a T-shaped type with respect to an inspection sample, and the center of the transducer element array B is located on an extension of a central line (not shown) penetrating the centers of the transducer elements a to n of the transducer element array A.

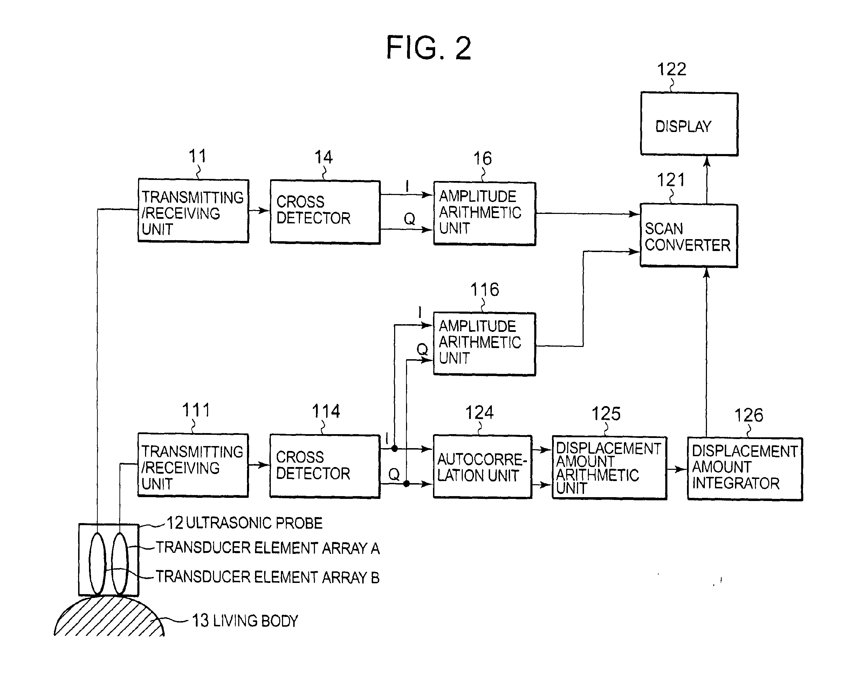

Fig. 2 shows an example of a block diagram of an ultrasonic diagnostic apparatus connected to the two transducer element arrays A, B. Fig. 2 differs from the apparatus of the conventional example shown in Fig. 15, in that a transmitting/receiving unit 11, an cross detector 14 and an amplitude arithmetic unit 16 are installed for the transducer element array B and the number of scan converters 121 is 3. The display 122 can display even the image of the amplitude information of the transducer element array B.

That is, in Fig. 2, an ultrasonic probe (hereafter, also merely referred to as a probe) 12 is provided with two transducer element arrays A, B. Electronic pulses of high voltages are outputted by the transmitting/receiving units 111, 11 to the transducer element arrays A, B, respectively, and converted into ultrasonic signals in the transducer element arrays A, B, and transmitted in a direction where the information of a living body 13 is desired to be obtained. The ultrasonic pulses of the transducer element arrays A, B are reflected in an organ (or a blood vessel or the like), in which the information inside the living body 13 is desired to be obtained, respectively and received by the transducer element arrays A, B and passed through the transmitting/receiving units 111, 11 and detected by cross detectors 114, 14 by using a reference signal of a frequency approximately equal to a transmission frequency, and two signals of I, Q are outputted.

The two signals of I, Q from the cross detectors 114, 14 are inputted to amplitude arithmetic units 116, 16, respectively, and converted into amplitude information, and those signals are used for the B-mode displaying. The signals of I, Q are also inputted to an autocorrelation unit 124. The autocorrelation unit 124 correlates the equal depths of the signals that are transmitted and received in the same direction two times and continuously determines a rotation amount of a phase. The rotation amount of the phase is proportional to the movement amount of the organ. It is a displacement amount arithmetic unit 125 that carries out this computation. The displacement amount computed by the displacement amount arithmetic unit 125 is integrated by a displacement amount integrator 126. Consequently, by totaling the micro motions from a certain time, it is possible to determine the position to which the organ moves. The B-mode images determined by the amplitude arithmetic units 116, 16 and the displacement amount determined by the displacement amount integrator 126 are displayed through the scan converter 121 on the display 122.

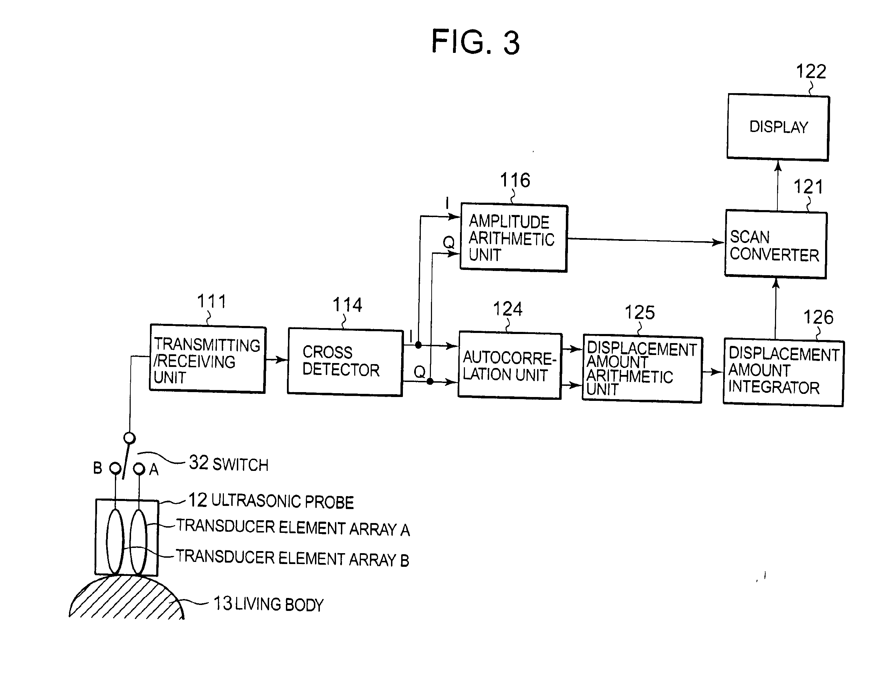

Fig. 3 is another block diagram of the ultrasonic diagnostic apparatus connected to the two transducer element arrays. In this embodiment, the circuits 11, 14 and 16 on the transducer element array B side shown in Fig. 2 are omitted, and a switch 32 can be used to switch to any of the transducer element arrays A, B.

The operations in this embodiment will be described below. Fig. 1B is a top view showing a situation that the probe 12 is placed on a blood vessel 4. At this point, an image of the transducer element array B is displayed on the display 122. An operating person adjusts a position of the probe 12 so that a center of a radius direction (round cutting direction) of the blood vessel 4 is located between the transducer elements e and f in the central portion of the transducer element array B. At this time, on the image, when an appropriate line is indicated between the transducer elements e and f, the adjustment is easy. At a stage when the adjustment is ended, as for the relation between the transducer element array B and the blood vessel 4, the respective centers are positioned as shown in the right side view of Fig. 1C.

Next, the operating person operates the probe 12 so that the transducer element array A is aligned with a longitudinal direction of the blood vessel 4. At this time, as shown in the top view of Fig. 1D, by circularly moving the transducer element array A (an array direction β) with a central point o of the transducer element array B as an axis, it can be aligned so as to be along a direction α of the blood vessel 4. In this work, the image of the transducer element array A is displayed. At this time, in view of the work, if the shape of the transducer element array B is convex, the circular operation can be carried out without any displacement of the central point o. As for the position relation between the transducer element array A and the blood vessel 4 when the positioning is ended, the respective directions are positioned as shown in the front view of Fig. 1E.

As mentioned above, the usage of the transducer element array B orthogonal to the transducer element array A enables the determination of one end of the transducer element array A. After that, with the circular operation, the transducer element array A can be easily aligned with the longitudinal direction of the blood vessel 4. Thus, the excellent image of the blood vessel can be obtained.

### <Second Embodiment>

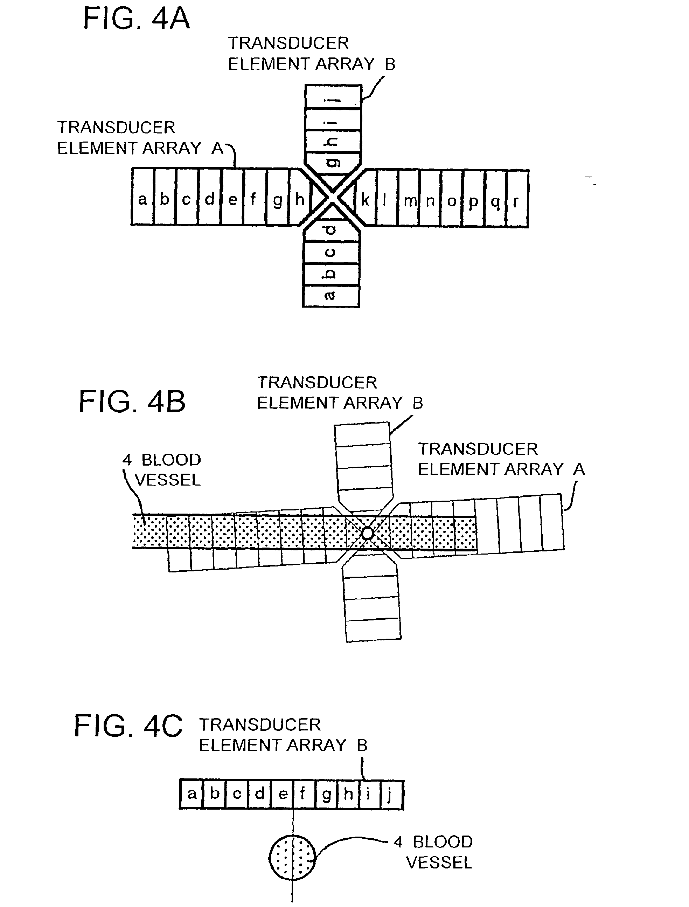

Figs. 4A to 4E are explanation views when inspection regions of a plurality of transducer element arrays in the second embodiment of the present invention are positioned to the blood vessel. Fig. 4A is a top view showing the arrangements of the two transducer element arrays A, B. Similarly to the first embodiment, the transducer element array A is provided with individual transducer elements a to r, and the transducer element array B is provided with individual transducer elements a to j. The transducer element array A and the transducer element array B are arranged in a cross-shaped type, as shown in Fig. 4A. A central line (not shown) penetrating the centers of the transducer elements a to r of the transducer element array A and a central line (not shown) penetrating the centers of the transducer elements a to j of the transducer element array B intersect squarely.

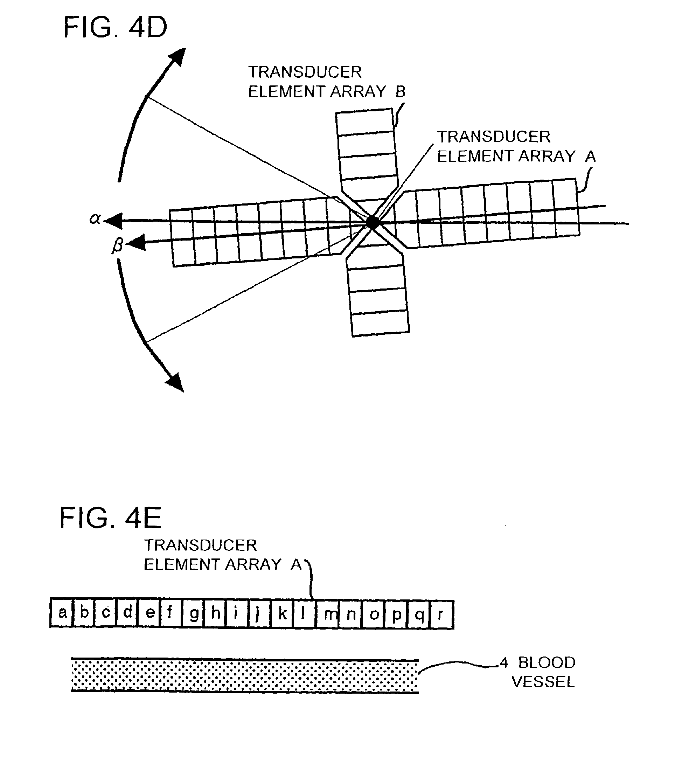

Even in the second embodiment of the present invention, Fig. 4B and Fig. 4C show the top view and the right side view, respectively. While the image of the transducer element array B is displayed, the center of the transducer element array B and the center of the blood vessel 4 are aligned. Next, with the intersection between the transducer element array A and the transducer element array B as a center, the transducer element array A is rotated as shown in the top view of Fig. 4D. Consequently, as shown in the front view of Fig. 4E, it can be aligned with the longitudinal direction of the blood vessel 4.

### <Third Embodiment>

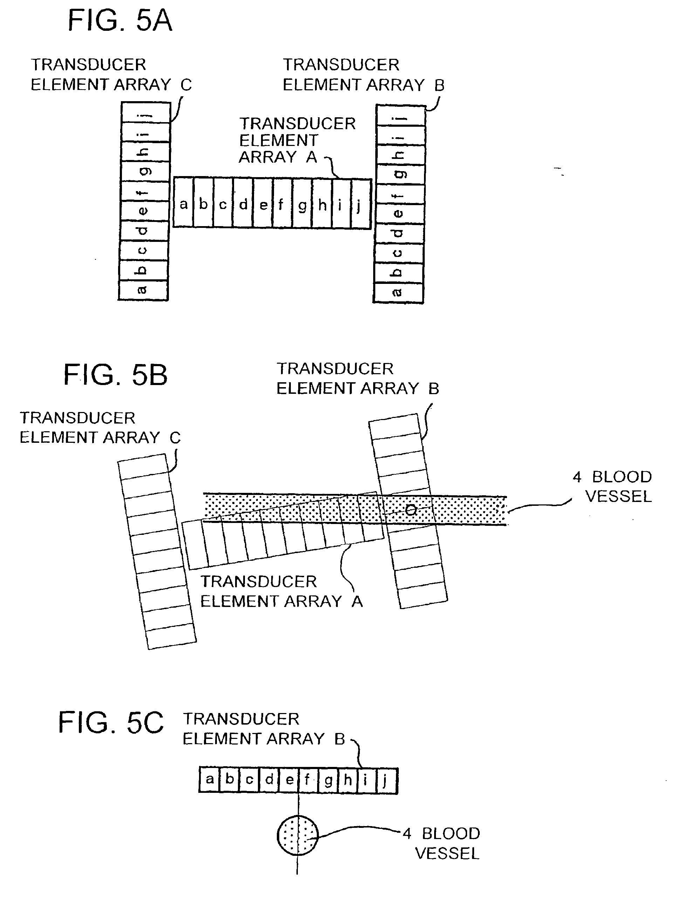

Figs. 5A to 5F are explanation views when inspection regions of a plurality of transducer element arrays in the third embodiment of the present invention are positioned to the blood vessel. This embodiment uses three transducer element arrays A, B and C. Fig. 5A is a top view showing the arrangements of the three transducer element arrays A, B and C. The transducer element array A is provided with individual transducer elements a to j, and the transducer element arrays B, C are provided with individual transducer elements a to j, respectively. The transducer element array A, the transducer element array B and the transducer element array C are arranged in an H-shaped type, as shown in Fig. 5A. An extension of a central line (not shown) penetrating the centers of the transducer elements a to j of the transducer element array A is located at the centers of the transducer element array B and the transducer element array C.

The ultrasonic diagnostic apparatus connected to the probe in the third embodiment of the present invention is approximately similar to Figs. 2, 3, and a circuit for the transducer element array C is added to Fig. 2, or the switch 32 of Fig. 3 is branched.

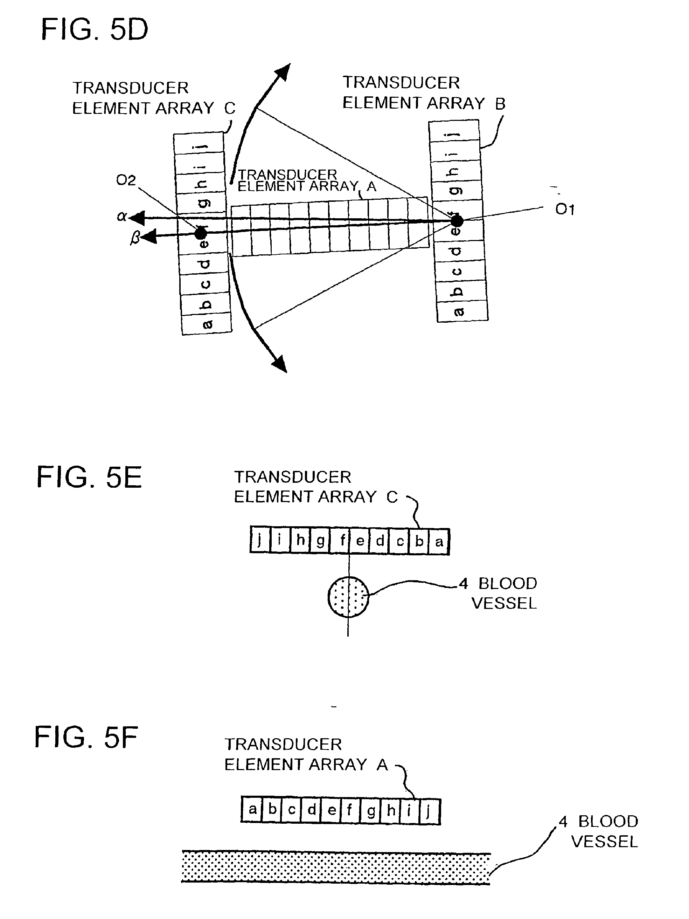

In this embodiment, at first, in the parallel transducer element arrays B, C, arbitrarily, for example, the transducer element array B is used to display its image, and the respective centers of the transducer element array B and the blood vessel 4 are aligned as shown in the top view and the right side view of Fig. 5B and Fig. 5C. Next, the image of the transducer element array C parallel to the transducer element array B is displayed. As shown in the top view of Fig. 5D, the probe is rotated with a center O1 of the transducer element array B as an axis. The centers of the transducer element array C and the blood vessel 4 are aligned as shown in the left side of Fig. 5E. At this time, the longitudinal directions of the transducer element array A and the blood vessel 4 are aligned as shown in the front view of Fig. 5F.

By the way, the arrangement of the transducer element array is not limited to the above-mentioned embodiments. It may be the situation of their combinations. For example, a ladder type may be used in which two or more T-shaped types and H-shaped types are combined.

### <Fourth Embodiment>

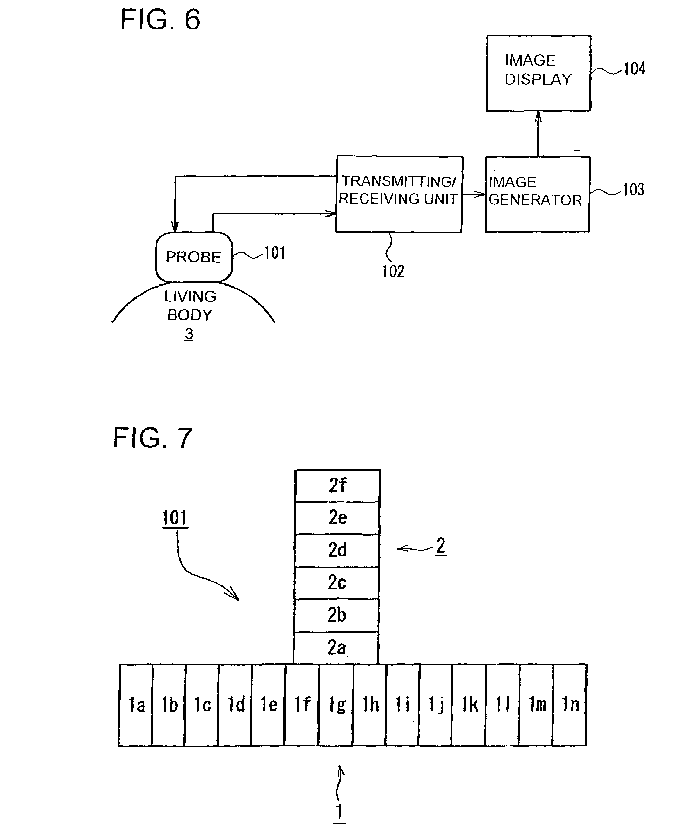

Fig. 6 is a configuration view showing one example of an ultrasonic diagnostic apparatus according to a fourth embodiment of the present invention. This ultrasonic diagnostic apparatus includes: a probe 101 for transmitting and receiving an ultrasonic signal to and from a living body 3; a transmitting/receiving unit 102 for transmitting and receiving an electric signal to and from the probe 101; an image generator 103 for generating a fault image in accordance with the electric signal received by the transmitting/receiving unit 102; and an image display 104 for displaying the fault image generated by the image generator 103.

The probe 101 transmits and receives the ultrasonic signal to and from the living body 3. Fig. 7 is a diagrammatic view showing one example of the configuration of the probe. This probe 101 includes a first transducer element array 1 and a second transducer element array 2. The first transducer element array 1 includes a plurality of transducer elements 1a to In, and the second transducer element array 2 includes a plurality of transducer elements 2a to 2f. Those transducer element arrays are arranged such that the array directions of the vibratos intersect each other. Also, preferably, the transducer element arrays are arranged so as not to overlap with each other. For example, in this embodiment, as illustrated, the first transducer element array 1 and the second transducer element array 2 are arranged so as to form a T-shaped type.

The operation of the ultrasonic diagnostic apparatus will be described below.

At first, the probe 101 is brought into contact with the living body 3 surface that is the target of an inspection. The transmitting/receiving unit 102 transmits an electric signal (transmission signal) to the transducer element array, and this transmission signal is converted into the ultrasonic signal by the transducer element array and transmitted to the living body 3. The ultrasonic signal transmitted to the living body 3 is reflected by an inspection sample (for example, an atheroma inside the blood vessel or the like) inside the living body 3. This reflection wave is received by the transducer element array, converted into the electric signal (reception signal) and transmitted to the transmitting/receiving unit 102. A proper process (for example, a detection, an amplification or the like) is performed on the reception signal by the transmitting/receiving unit 102, and its output is inputted to the image generator 103. The transmitting/receiving operation is repeated while the ultrasonic wave is scanned in the transducer element array.

The foregoing operation is performed on the first transducer element array 1 and the second transducer element array 2, respectively. At this time, an ultrasonic scanning method is assumed to be linear scanning in the first transducer element array 1 and oblique scanning in the second transducer element array 2. Here, [Oblique Scanning] implies the scanning for transmitting and receiving the ultrasonic wave that travels obliquely with regard to the transmission/reception surface (the surface in contact with or opposite to the living body 3 surface) of the transducer element array.

In succession, the image generator 103 generates a first fault image of the inspection sample in accordance with the reception signal obtained about the first transducer element array 1 and generates a second fault image of the inspection sample in accordance with the reception signal obtained about the second transducer element array 2. The image generating method is not especially limited. For example, a digital scan conversion method and the like can be employed. Then, the first fault image and second fault image which are generated by the image generator 103 are displayed on the image display 104. At this time, a guide line indicating a central position of each transducer element array, together with the fault image of the inspection sample is preferred to be displayed on the image display 104.

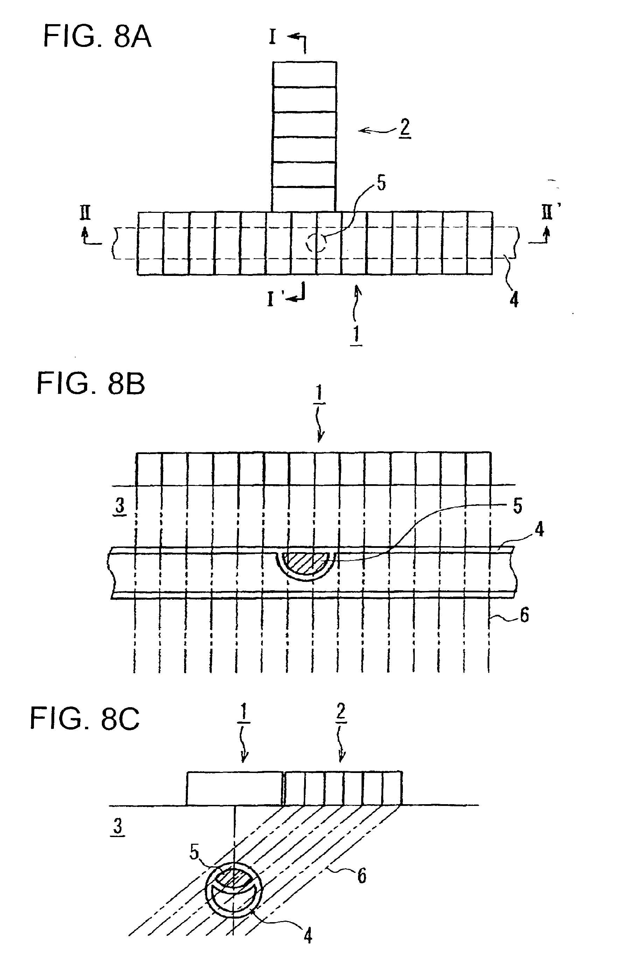

Moreover, the positioning between the probe and the inspection sample is carried out in accordance with the first fault image and second fault image which are displayed on the image display 104. The positioning of this probe is explained by exemplifying the case that the inspection sample is the atheroma generated inside the blood vessel. Fig. 8A to Fig. 8C are diagrammatic views showing the position relation between the first and second transducer element arrays and the inspection sample when the positioning of this probe is executed. Fig. 8A corresponds to a top view, Fig. 8B corresponds to its II-II' section view, and Fig. 8C corresponds to the I-I' section view.

As shown in Fig. 8A to 8C, the positioning of the probe is carried out such that the blood vessel 4 is located immediately under the first transducer element array 1, and the blood flow direction agrees with the array direction of the first transducer element array 1. Then, the positioning is carried out such that the atheroma 5 generated inside the blood vessel 4 is located immediately under the first transducer element array 1 and on an extension line of a central line extending in the array direction of the second transducer element array 2.

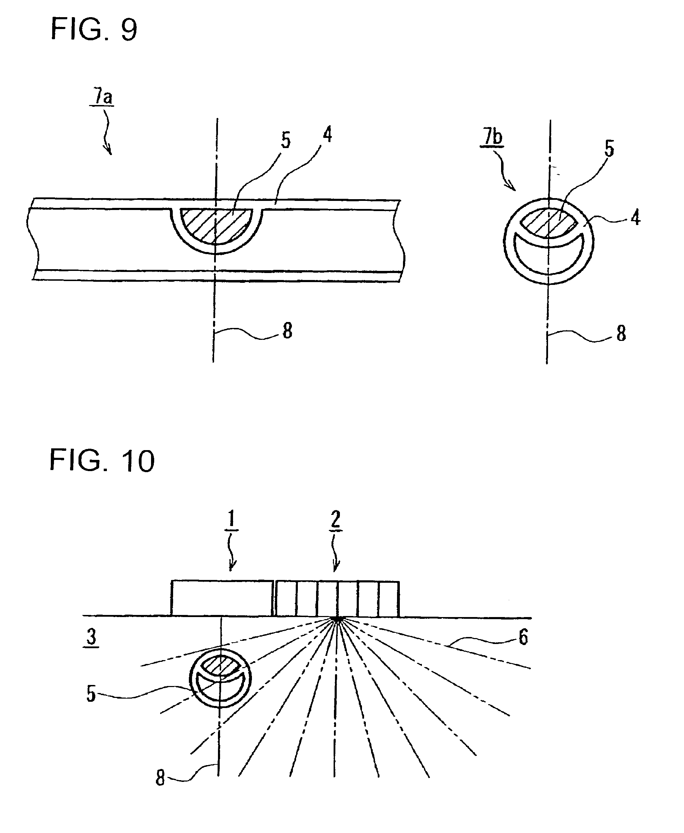

Fig. 9 is a view showing one example of a screen displaying after the positioning. In this way, according to the foregoing ultrasonic diagnostic apparatus, a longitudinal section of the blood vessel 4 (a section including a central axis of the blood vessel 4) is displayed as a first fault image 7a, and a lateral section of the blood vessel 4 (a section orthogonal to the central axis of the blood vessel 4) is displayed as a second fault image 7b. Then, on both of the fault images, the guide line 8 is aligned with the position of the atheroma 5. In this way, the foregoing positioning can be executed by aligning the guide line 8 displayed on the image display 104 with the position of the atheroma 5 in the fault image.

In this way, according to the foregoing ultrasonic diagnostic apparatus, the probe includes the plurality of transducer element arrays which are arranged such that the array directions intersect each other, and the plurality of fault images corresponding to the respective transducer element arrays can be displayed. Thus, since the positions of the probe and the inspection sample can be checked from at least two directions, the position of the probe can be aligned with the position of the inspection sample easily and surely. As a result, the signal corresponding to the inspection sample can be obtained at the excellent reproducibility.

In particular, in this embodiment, as shown in Fig. 8C, the second transducer element array 2 carries out the oblique scanning. When the second transducer element array 2 does not carry out the oblique scanning and it is assumed to carry out the usual linear scanning (the scanning for transmitting/receiving the ultrasonic wave which travels approximately vertical to the transmission/reception surface of the transducer element array), if the fault image of the inspection sample is tried to be obtained in the second transducer element array, the inspection sample needs to exist immediately under the second transducer element array. Thus, in this case, the intersection between the first transducer element array and the second transducer element array is required, which results in a problem of the shape of the transducer element in the intersection portion. For example, if the widths of the mutual transducer elements in the intersection portion are made narrower, there may be a fear that the sensibility drop in the portion occurs.

On the contrary, when the second transducer element array 2 carries out the oblique scanning, even if the inspection sample is not located immediately under the second transducer element array 2, the fault image of the inspection sample can be obtained by the second transducer element array 2. Thus, without any intersection between the first transducer element array 1 and the second transducer element array 2, for example, they can be arranged in the T-shaped type. Hence, the foregoing sensibility drop can be suppressed.

By the way, in the foregoing explanation, the case that the second transducer element array 2 carries out the oblique scanning is exemplified. However, as shown in Fig. 10, the second transducer element array 2 may carry out a sector scanning. Similarly to the foregoing case, even with such manner, even if the inspection sample is not located immediately under the second transducer element array, the fault image of the inspection sample can be obtained by the second transducer element array. Thus, the first transducer element array 1 and the second transducer element array 2 can be arranged without any intersection between them.

### <Fifth Embodiment>

One example of an ultrasonic diagnostic apparatus according to the fifth embodiment of the present invention will be described below. This ultrasonic diagnostic apparatus includes the probe, the transmitting/receiving unit, the image generator and the image display, similarly to the fourth embodiment.

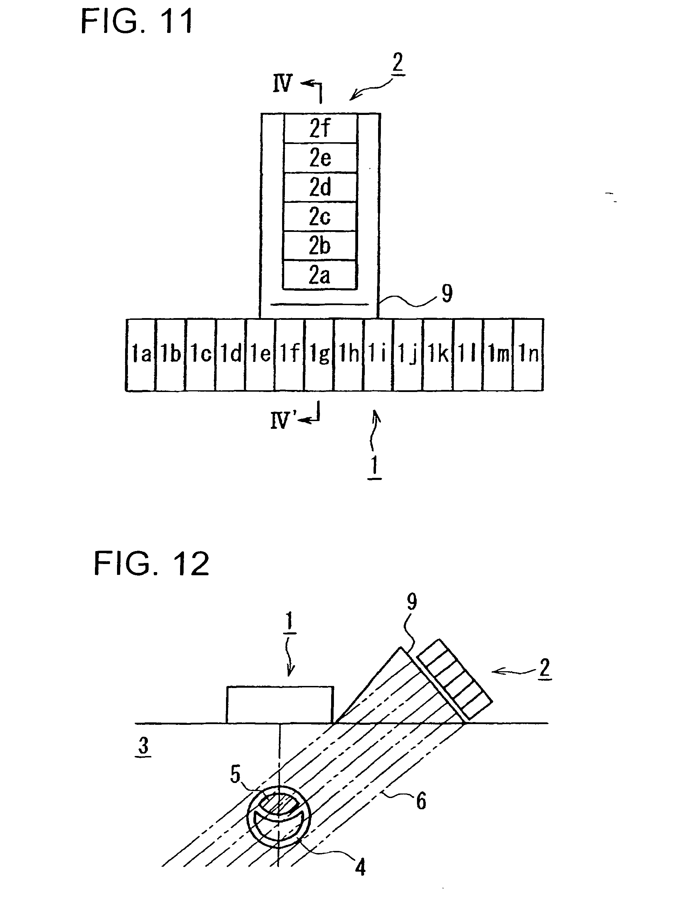

Fig. 11 is a diagrammatic view showing one example of the configuration of the probe in this embodiment. Also, Fig. 12 is a diagrammatic view showing the probe when the ultrasonic diagnostic apparatus is used, and corresponds to the IV-IV' section view of Fig. 11.

This probe includes the first transducer element array 1 and the second transducer element array 2. The first transducer element array 1 includes a plurality of transducer elements 1a to In, and the second transducer element array 2 includes a plurality of transducer elements 2a to 2f. These transducer element arrays are arranged such that the array directions of the transducer elements intersect each other, similarly to the fourth embodiment.

In this embodiment, a transmission/reception surface of the second transducer element array 2 is inclined to a transmission/reception surface of the first transducer element array 1. In other words, the second transducer element array 2 is arranged such that the ultrasonic transmission/reception surface is inclined to the living body 3 surface, as shown in Fig. 12, when the ultrasonic diagnostic apparatus is used.

Such arrangement can be attained by placing the second transducer element array 2 on a pedestal 9, as illustrated. As the pedestal 9, for example, the component where a medium is filled in a sump can be used. In this case, preferably, the sump has flexibility and can be freely deformed depending on the shape of the living body 3 surface, so as to be able to adhere to the living body 3 surface. The material constituting the sump and medium is not especially limited, unless it does not impede the transmission of the ultrasonic wave. For example, as the sump, silicon rubber, urethane rubber and the like can be used. As the medium, hydrated gelatin and the like can be used.

By the way, the operation of the foregoing ultrasonic diagnostic apparatus is similar to the fourth embodiment. However, in this embodiment, as shown in Fig. 12, the second transducer element array 2 carries out the linear scanning.

Even in the foregoing ultrasonic diagnostic apparatus, similarly to the fourth embodiment, the positions of the probe and the inspection sample can be checked from at least two directions. Thus, the position of the probe can be aligned with the position of the inspection sample easily and surely. As a result, the signal corresponding to the inspection sample can be obtained at the excellent reproducibility.

Also, as mentioned above, in this embodiment, the second transducer element array 2 carries out the linear scanning. The second transducer element array 2 is arranged such that the transmission/reception surface is inclined to the living body 3 surface. Thus, when this second transducer element array carries out the linear scanning, the ultrasonic wave that travels obliquely to the living body 3 surface is transmitted and received. Hence, the effect similar to the case when the second transducer element array carries out the oblique scanning can be obtained as explained in the fourth embodiment.

### <Sixth Embodiment>

One example of an ultrasonic diagnostic apparatus according to the sixth embodiment of the present invention will be described below. This ultrasonic diagnostic apparatus includes the probe, the transmitting/receiving unit, the image generator and the image display, similarly to the fourth embodiment.

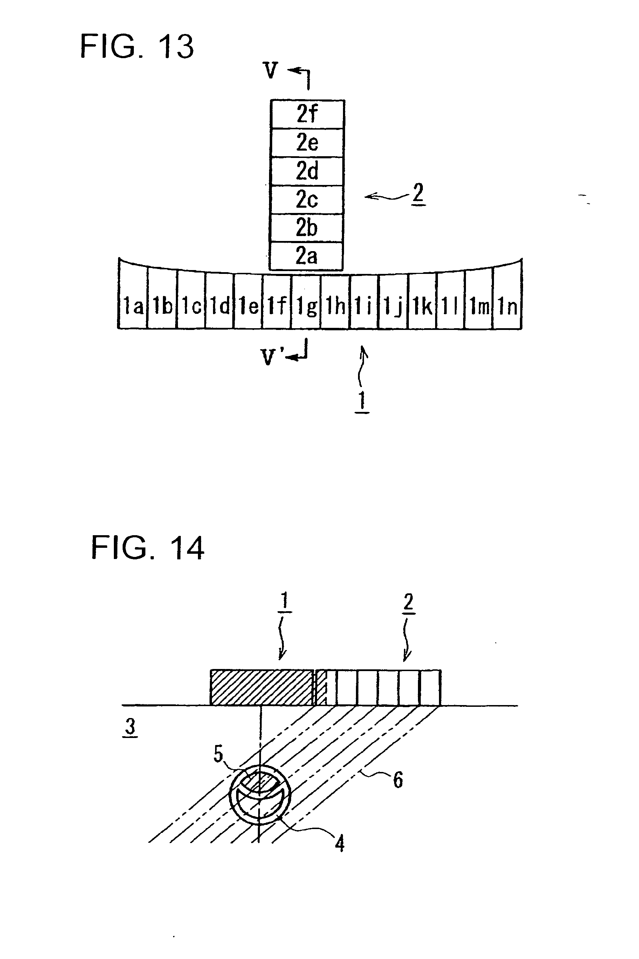

Fig. 13 is a diagrammatic view showing one example of the configuration of the probe in this embodiment. Also, Fig. 14 is a diagrammatic view showing the probe when the ultrasonic diagnostic apparatus is used, and corresponds to the V-V' section view of Fig. 13. Incidentally, the oblique line portion of Fig. 14 indicates the projection of the portion away from the second transducer element array 2 of the first transducer element array 1 (the portion corresponding to the transducer element 1a or In of Fig. 13).

This probe includes the first transducer element array 1 and the second transducer element array 2. The first transducer element array 1 includes a plurality of transducer elements 1a to In, and the second transducer element array 2 includes a plurality of transducer elements 2a to 2f. These transducer element arrays are arranged such that the array directions of the transducer elements intersect each other, similarly to the fourth embodiment. For example, in this embodiment, as illustrated, the first transducer element array 1 and the second transducer element array 2 are arranged so as to constitute the T-shaped type.

In this embodiment, as shown in Fig. 13, the width of the first transducer element array 1 is adjusted so as to be small in the portion close to the second transducer element array 2. Here, [Width of Transducer Element Array] implies the dimension in the direction that is parallel to the flat surface generated by the two transducer element arrays and orthogonal to the array direction. The drop in the width in the portion close to this second transducer element array is preferably attained by depressing the end surface of the second transducer element array 2 side of the first transducer element array 1 against the central line side of the width of the first transducer element array 1. In this case, the end surface of the first transducer element array 1 can be depressed, for example, up to about 70% of the width of the 1a portion, preferably up to about 75%, in the portion close to the second transducer element array 2. Also, in order to protect the deterioration in ultrasonic wave image quality, the width of the first transducer element array 1 is desired to be 4mm or more even in the minimum portion, for example, in the probe having a frequency of 7MHz.

By the way, the operation of the foregoing ultrasonic diagnostic apparatus is similar to the fourth embodiment, and the ultrasonic scanning in the second transducer element array 2 is carried out by the oblique scanning or sector scanning.

Even in the foregoing ultrasonic diagnostic apparatus, similarly to the fourth embodiment, the positions of the probe and the inspection sample can be checked from at least two directions. Thus, the position of the probe can be aligned with the position of the inspection sample easily and surely. As a result, the signal corresponding to the inspection sample can be obtained at the excellent reproducibility.

Also, as mentioned above, in this embodiment, the width of the first transducer element array is thin in the portion close to the second transducer element array. Thus, as shown in Fig. 14, as compared with the fourth embodiment, the position of the second transducer element array 2 is the position close to the inspection sample. Consequently, when the second transducer element array carries out the oblique scanning, the angle in the traveling direction of the ultrasonic wave with respect to the living body 3 surface can be made large (made close to the direction vertical to the living body 3 surface). As a result, the image quality of the obtained fault image can be improved. Also, even if the second transducer element array carries out the sector scanning, the inspection sample can be scanned in the range where the deflection of an ultrasonic beam is little. Hence, the excellent image quality can be obtained.

As evident from the foregoing first to third embodiments, the present invention can provide the ultrasonic diagnostic apparatus, which uses the probe having the two or more transducer element arrays, and firstly uniforms the round cutting direction of the blood vessel with one probe, and next operates the other probe orthogonal to this in the circular manner, and can consequently uniform the longitudinal direction of the blood vessel and the direction of the transducer element, can obtain the image having the excellent image quality, can position to the straight organ such as the blood vessel and observe without any skilled technique, and can obtain the motion information at the high precision.

In the present invention, as evident from the foregoing fourth to sixth embodiments, the probe includes the plurality of transducer element arrays, and the plurality of fault images are displayed correspondingly to the reception signals of the respective transducer element arrays. Thus, the positions of the probe and the inspection sample can be checked from the plurality of different directions. Hence, the present invention can provide the ultrasonic diagnostic apparatus that can align the position of the probe with the position of the inspection sample accurately at the excellent reproducibility.

### INDUSTRIAL APPLICABILITY

The present invention provides the ultrasonic diagnostic apparatus in which the positioning is easy and the excellent image can be obtained. The present invention is useful for the medical practice for carrying out a diagnosis and a medical action and also useful for developing and manufacturing the medical equipment including the ultrasonic diagnostic apparatus.

## Claims

1. An ultrasonic diagnostic apparatus, including; a plurality of transducer element arrays joined at any angle; and means for displaying images respectively obtained from each of said plurality of transducer element arrays, wherein said transducer element array is configured by arraying a plurality of transducer elements in a parallel state.

2. The ultrasonic diagnostic apparatus according to claim 1, wherein array directions of said plurality of transducer element arrays are arranged so as to be orthogonal.

3. The ultrasonic diagnostic apparatus according to claim 1, wherein in said plurality of transducer element arrays, two transducer element arrays are arranged in a T-shaped type.

4. The ultrasonic diagnostic apparatus according to claim 1, wherein in said plurality of transducer element arrays, two transducer element arrays are arranged in a cross-shaped type.

5. The ultrasonic diagnostic apparatus according to claim 1, wherein in said plurality of transducer element arrays, three transducer element arrays are arranged in an H-shaped type.

6. An ultrasonic diagnostic apparatus, **characterized by** including: a probe for transmitting an ultrasonic wave into a living body and receiving a reflection wave from an inspection sample inside said living body; an image generator for generating a fault image of said inspection sample in accordance with a signal received by said probe; and an image display for displaying said fault image,
wherein said probe has a first transducer element array and a second transducer element array which are arranged such that array directions of transducer elements intersect each other, and
said image generator and said image display generate and display a first fault image corresponding to a signal received by said first transducer element array and a second fault image corresponding to a signal received by said second transducer element array.

7. The ultrasonic diagnostic apparatus according to claim 6, wherein said image display displays a guide line that indicates positions of said first transducer element array and said second transducer element array, together with a fault image of an inspection sample.

8. The ultrasonic diagnostic apparatus according to claim 6, wherein said first transducer element array and said second transducer element array are arranged so as not to overlap with each other.

9. The ultrasonic diagnostic apparatus according to claim 8, wherein said first transducer element array carries out a linear scanning.

10. The ultrasonic diagnostic apparatus according to claim 9, wherein said second transducer element array transmits and receives an ultrasonic wave that travels obliquely to a living body surface.

11. The ultrasonic diagnostic apparatus according to claim 9, wherein said second transducer element array carries out a sector scanning.

12. The ultrasonic diagnostic apparatus according to claim 8, wherein a width of said first transducer element array is adjusted so as to be small in a portion close to said second transducer element array.

13. The ultrasonic diagnostic apparatus according to claim 6, wherein an inspection sample is an atheroma existing inside a blood vessel.
